(19)

Europäisches
Patentamt
European
Patent Office
Office européen
des brevets

(11) **EP 4 094 682 A1**

(12) **EUROPEAN PATENT APPLICATION**

(43) Date of publication:
**30.11.2022 Bulletin 2022/48**

(21) Application number: **21176015.2**

(22) Date of filing: **26.05.2021**

(51) International Patent Classification (IPC):
**A61B 5/021** $^{(2006.01)}$ **A61B 5/00** $^{(2006.01)}$
**A61B 5/024** $^{(2006.01)}$

(52) Cooperative Patent Classification (CPC):
**A61B 5/7239; A61B 5/02108; A61B 5/02416;
A61B 5/7221**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventor: **DERKX, Rene Martinus Maria
5656 AE Eindhoven (NL)**

(74) Representative: **Philips Intellectual Property &
Standards
High Tech Campus 52
5656 AG Eindhoven (NL)**

(54) **METHOD, APPARATUS AND COMPUTER PROGRAM PRODUCT FOR ANALYSING A PULSE
WAVE SIGNAL**

(57) According to an aspect, there is provided a computer-implemented method for analysing a pulse wave signal, PWS, obtained from a subject. The PWS comprises pulse wave measurements for one or more cardiac cycles of the subject during a first time period. The method comprises (i) determining (101) 1PWS as a first derivative with respect to time of the PWS; (ii) determining (103) 2PWS as a second derivative with respect to time of the PWS; (iii) analysing (105) the 2PWS to identify a first time point corresponding to the occurrence of a first maximum value in the 2PWS; (iv) determining (107) a value of the 1PWS at the first time point; (v) normalising (109) the value of the 1PWS at the first time point with respect to a maximum value of the 1PWS; (vi) evaluating (111) the quality of the PWS using the normalised value.

Determine 1PWS as a first derivative
with respect to time of the PWS — 101

Determine 2PWS as a second derivative
with respect to time of the PWS — 103

Analyse the 2PWS to identify a first
time point corresponding to the occurrence
of a first maximum value in the 2PWS — 105

Determine a value of the 1PWS
at the first time point — 107

Normalise the value of the 1PWS
at the first time point with respect to a
maximum value of the 1PWS — 109

Evaluate the quality of the PWS
using the normalised value — 111

Fig. 8

EP 4 094 682 A1

**Description**

FIELD OF THE INVENTION

This disclosure relates to pulse wave signals obtained from a subject, with the pulse wave signal comprising pulse wave measurements for one or more cardiac cycles of the subject during a time period. More particularly, this disclosure relates to a method, apparatus and a computer program product for analysing pulse wave signals.

BACKGROUND OF THE INVENTION

In many haemodynamic monitoring applications, the arterial pulse is one of the most important physiological processes to be measured in order to derive haemodynamic parameters. For example, in invasive blood pressure monitoring, a catheter inside the artery of a subject can measure the arterial pulse, blood pressure, etc. This measurement is known as the measurement of arterial blood pressure (ABP). From this arterial pulse, also other important haemodynamic parameters can be computed, for example cardiac output, stroke volume, etc. Another example is the measurement of pulsations from the outside of the artery of a subject, e.g. via photoplethysmography (PPG) measurements. An important use-case for PPG measurements is as a surrogate blood pressure measurement, i.e. a measurement of a physiological characteristic from which blood pressure, or changes in blood pressure, can be derived. Furthermore, PPG signals can also be used to measure the oxygen concentration in the blood, known as a $SpO_2$ measurement. This is done by measuring PPG signals for two wavelengths of light, e.g. red and infrared. From these two signals, the $SpO_2$ can be determined as a metric for the oxygenation of the blood.

A PPG signal is an example of a pulse wave signal (PWS), i.e. a signal representing measurements of a pulse wave. In some applications, signals such as PPG signals can be corrupted by motion artefacts, for example where a subject wearing a PPG sensor is doing physical activities or exercise. Alternatively, in medical applications, such as during surgery, the perfusion of the blood in the part of the body where the PPG sensor is being worn (e.g. a peripheral body part such as a finger) can diminish or reduce (because of, for example, the application of medication or physiological blood-pressure regulation effects by smooth muscles) and the signal-to-noise ratio of the useful signal in the measured PPG signal (e.g. measured via PPG methods) can drop such that metrics computed from the PPG signal are less accurate.

However, it may not be immediately clear, or evident at all, that the quality of the PPG signal or other PWS has degraded (temporarily or otherwise), or is otherwise unable to provide a reliable measure of the monitored physiological characteristic (e.g. heart rate, $SpO_2$, blood pressure, etc.).

SUMMARY OF THE INVENTION

Some techniques are known in the art for computing a measure of the quality of measured arterial pulses, for example as described in "Signal quality measure for pulsatile physiological signals using morphological features: Applications in reliability measure for pulse oximetry" by Elyas Sabeti, et.al., Informatics in Medicine Unlocked, Volume 16, 2019. In one technique, the 'skewness' of the measured signal was used as a metric for assessment of PPG quality. However, often such general metrics need to be tuned to specific sensors and sensor front-ends, e.g. filtering for controlling the effective bandwidth of the signal. Furthermore, the metrics are often sensitive to arterial compliance variations. It is known that subjects with higher ages, vascular complications (e.g. stiff arteries) or smooth-muscle contractions will lower the vascular compliance.

Therefore, there is a need for improved techniques for assessing the quality of a PWS that provide straightforward quality measures and that are independent of the sensor characteristics and/or has a lowered dependency for vascular stiffness, making the technique more robust when compared to known techniques in the art.

According to a first specific aspect, there is provided a computer-implemented method for analysing a pulse wave signal, PWS, obtained from a subject. The PWS comprises pulse wave measurements for one or more cardiac cycles of the subject during a first time period. The method comprises (i) determining 1PWS as a first derivative with respect to time of the PWS; (ii) determining 2PWS as a second derivative with respect to time of the PWS; (iii) analysing the 2PWS to identify a first time point corresponding to the occurrence of a first maximum value in the 2PWS; (iv) determining a value of the 1PWS at the first time point; (v) normalising the value of the 1PWS at the first time point with respect to a maximum value of the 1PWS; and (vi) evaluating the quality of the PWS using the normalised value. Thus, this technique provides a straightforward quality measure that is independent of the sensor characteristics and/or has a lowered dependency for vascular stiffness.

In some embodiments, the first maximum value in the 2PWS and/or the value of the 1PWS at the first time point and/or the maximum value of the 1PWS are determined from a plurality of sample values of the 2PWS and/or 1PWS. These embodiments improve the accuracy of evaluation of the quality of the PWS.

In some embodiments, step (iii) comprises identifying the first maximum value in the 2PWS as the maximum value of the 2PWS in a portion of the 2PWS, wherein the portion of the 2PWS has a duration equal to or less than the duration of a cardiac cycle.

In some embodiments, step (vi) comprises determining the quality of the PWS according to a difference between the normalised value and a target value, T.

In some embodiments, wherein step (vi) comprises determining a quality indicator, QI, according to:

$$QI = e^{-\left(\frac{Q-T}{s}\right)^2},$$ where Q is the normalised value and s is a sensitivity value representing acceptable deviation of Q from the target value, T.

In these embodiments, the target value, T, may 0.5. In alternative embodiments, the target value, T, depends on one or more characteristics of the subject. These latter embodiments enable the target value to be set or varied between subjects.

In some embodiments, the method further comprises performing at least one repetition of steps (iii)-(v) for at least a second time point corresponding to the occurrence of at least a second maximum value in the 2PWS; and step (vi) comprises evaluating the quality of the PWS using the normalised values. These embodiments enable the quality of the PWS to be evaluated over time. In these embodiments, step (vi) can comprise evaluating the quality of the PWS using a function of the normalised values. In these embodiments, the function of the normalised values can be a smoothing function. In these embodiments, the first maximum value can relate to a first cardiac cycle in the first time period and the second maximum value can relate to a second cardiac cycle in the first time period.

In some embodiments, the method further comprises obtaining the PWS from a pulse wave sensor.

In some embodiments, the method further comprises obtaining a sensor signal from a pulse wave sensor, wherein the sensor signal comprises pulse wave measurements of the subject by the pulse wave sensor according to a first sampling rate; and generating the PWS by using an interpolation technique on the sensor signal to increase the sampling rate. Increasing the sampling rate can improve the reliability or accuracy of the quality evaluation.

In some embodiments, the method further comprises determining whether to compute a physiological characteristic for the subject from the PWS according to the quality of the PWS.

In some embodiments, the method further comprises outputting an indication of the quality or reliability of the PWS according to the evaluated quality.

According to a second aspect, there is provided a computer program product comprising a computer readable medium having computer readable code embodied therein. The computer readable code is configured such that, on execution by a suitable computer or processor, the computer or processor is caused to perform the method according to the first aspect or any embodiment thereof.

According to a third specific aspect, there is provided an apparatus configured to analyse a pulse wave signal, PWS, obtained from a subject. The PWS comprises pulse wave measurements for one or more cardiac cycles of the subject during a first time period. The apparatus comprises a processing unit configured to (i) determine 1PWS as a first derivative with respect to time of the PWS; (ii) determine 2PWS as a second derivative with respect to time of the PWS; (iii) analyse the 2PWS to identify a first time point corresponding to the occurrence of a first maximum value in the 2PWS; (iv) determine a value of the 1PWS at the first time point; (v) normalise the value of the 1PWS at the first time point with respect to a maximum value of the 1PWS; and (vi) evaluate the quality of the PWS using the normalised value. Thus, this technique provides a straightforward quality measure that is independent of the sensor characteristics and/or has a lowered dependency for vascular stiffness.

In some embodiments, the first maximum value in the 2PWS and/or the value of the 1PWS at the first time point and/or the maximum value of the 1PWS are determined from a plurality of sample values of the 2PWS and/or 1PWS. These embodiments improve the accuracy of evaluation of the quality of the PWS.

In some embodiments, in operation (iii), the processing unit is configured to identify the first maximum value in the 2PWS as the maximum value of the 2PWS in a portion of the 2PWS, wherein the portion of the 2PWS has a duration equal to or less than the duration of a cardiac cycle.

In some embodiments, in operation (vi) the processing unit is configured to determine the quality of the PWS according to a difference between the normalised value and a target value, T. In some embodiments, in operation (vi) the processing unit is configured to determine a quality indicator, QI, according to: $QI = e^{-\left(\frac{Q-T}{s}\right)^2}$, where Q is the normalised value and s is a sensitivity value representing acceptable deviation of Q from the target value, T.

In these embodiments, the target value, T, may 0.5. In alternative embodiments, the target value, T, depends on one or more characteristics of the subject. These latter embodiments enable the target value to be set or varied between subjects.

In some embodiments, the processing unit is further configured to perform at least one repetition of operations (iii)-(v) for at least a second time point corresponding to the occurrence of at least a second maximum value in the 2PWS;

and in operation (vi) the processing unit is configured to evaluate the quality of the PWS using the normalised values. These embodiments enable the quality of the PWS to be evaluated over time. In these embodiments, in operation (vi) the processing unit can be configured to evaluate the quality of the PWS using a function of the normalised values. In these embodiments, the function of the normalised values can be a smoothing function. In these embodiments, the first maximum value can relate to a first cardiac cycle in the first time period and the second maximum value can relate to a second cardiac cycle in the first time period.

In some embodiments, the apparatus is further configured to obtain the PWS from a pulse wave sensor.

In some embodiments, the apparatus is further configured to obtain a sensor signal from a pulse wave sensor, wherein the sensor signal comprises pulse wave measurements of the subject by the pulse wave sensor according to a first sampling rate; and the processing unit is further configured to generate the PWS by using an interpolation technique on the sensor signal to increase the sampling rate. Increasing the sampling rate can improve the reliability or accuracy of the quality evaluation.

In some embodiments, the processing unit is further configured to determine whether to compute a physiological characteristic for the subject from the PWS according to the quality of the PWS.

In some embodiments, the processing unit is further configured to output an indication of the quality or reliability of the PWS according to the evaluated quality.

These and other aspects will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1(a) shows a 1-second portion of a PPG signal, and Figs 1(b) and 1(c) show the first and second derivatives of the PPG signal respectively;

Fig. 2 is a graph illustrating quality values, *Q,* for a patient over time derived according to various embodiments;

Fig. 3 is a graph illustrating a smoothed quality indicator, *QI,* for the patient shown in Fig. 2 derived according to various embodiments;

Fig. 4 is a graph illustrating a distribution of the quality value, *Q,* for the patient shown in Fig. 2;

Fig. 5 is a graph illustrating a distribution of quality values, *Q,* for a dataset including a number of different patients;

Fig. 6 is a graph illustrating the largest peak in quality value, *Q,* versus age of the patients;

Fig. 7 is a block diagram of an apparatus according to various exemplary embodiments;

Fig. 8 is a flow chart illustrating a method for analysing a pulse wave signal obtained from a subject to determine an indication of the blood pressure or a change in blood pressure; and

Fig. 9 is a functional block diagram illustrating various operations in analysing a pulse wave signal according to various embodiments.

DETAILED DESCRIPTION OF EMBODIMENTS

The techniques described herein provide for the assessment of the quality of a pulse wave signal, PWS. A PWS includes information about pulse changes/pulse waves at a measurement point on a body of a subject. The PWS can be, e.g., a PPG signal, or a pulse wave signal obtained using tonometry or a pressure sensor. According to the described techniques, the quality of the PWS is assessed using information derived from first-order and second-order derivatives of the PWS. The use of the derivatives is beneficial as any signal baseline (offset) is removed in such derivative signals. Offsets can occur, for example, due to characteristics of the sensor front-end, and are not related to the dynamic behaviour of the pulsating pressure-waves inside the artery.

In the following, the signal corresponding to the first derivative of the PWS with respect to time is denoted '1PWS' and the signal corresponding to the second derivative of the PWS with respect to time is denoted '2PWS'. When described with reference to the specific example of a PPG signal, the 1PWS is also referred to as a 'velocity waveform' (v-PPG) and the 2PWS is also referred to as an 'acceleration waveform' (a-PPG).

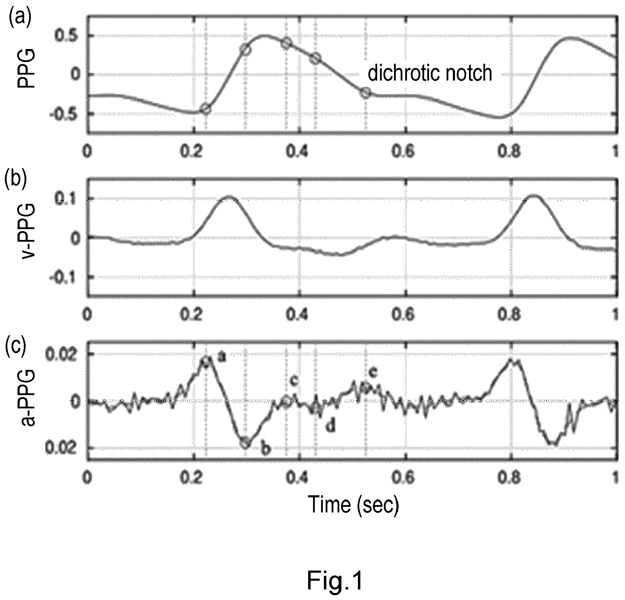

Fig. 1(a) shows an exemplary 1-second portion of a PPG signal with the timing of the dicrotic notch indicated. Fig. 1(b) shows the first derivative of the PPG signal in Fig. 1(a) with respect to time, and Fig. 1(c) shows the second derivative of the PPG signal in Fig. 1(a) with respect to time. Typically, it is assumed that the acceleration waveform consists of five 'fiducial points' or 'reference points', as shown in Fig 1. The five reference points are shown in the acceleration waveform, Fig 1(c). The *a* point marks the start of the direct pulse wave, which is the onset of the pulse wave (i.e. the pulse of blood caused by the beat of the heart) and the *b* point marks the end of the direct pulse wave. The *e* point marks the end of the systolic period (aortic valve closure), and for the purposes of this disclosure it is assumed that the c point marks the start of the reflected wave and the d point marks the end of the reflected wave.

In the following, the quality of the PWS is evaluated based on the value of the 1PWS at the occurrence of the a point in the second derivative of the PWS (a-PPG in Fig. 1) and the value of the peak of the first derivative of the PWS (v-PPG in Fig. 1). In particular, the time-value of the maximum of the second derivative of the PWS is used to evaluate the sample value in the first derivative of the PWS and this value is then normalised with the peak value of the first derivative of the PWS to obtain a metric that is independent of sensor scaling, etc. Experiments with actual patients measured during surgery and in the recovery room of a hospital show that this normalised value equals (ideally) 0.5, even under variations of arterial stiffness properties, e.g. arterial compliance variations due to the age of the patient.

It will be appreciated that the better the signal quality of the PWS/PPG, the less noise there will be in the a-PPG signal. The PPG signal in Fig. 1(a) has a sampling rate of 125 Hz with a 12-bit resolution. It can be seen that computing a second derivative of this PPG signal leads to a very poor signal because of the noise and quantisation in the PPG signal. Signal smoothing (either temporal or over multiple heart cycles) can be applied in order to have improved fiducial point detections. A smoothed signal is overlaid on the a-PPG in Fig. 1(c), and this smoothed signal was obtained by applying a Savitz-Golay filter (a polynomial smoothing filter) to the a-PPG signal, with a polynomial order of 4 and a frame-length 11.

In some embodiments, to improve the time resolution of the peak detections, interpolation techniques can be used that assume a limited bandwidth of the PWS/PPG signal and estimate time values and sample values in between two samples of the original PWS/PPG signal.

As noted above, the quality of the PWS can be given by the sample value in the first derivative of the PWS coinciding with the maximum of the second derivative of the PWS (the a point) normalised with the peak value of the first derivative of the PWS. This quality value, which is denoted Q, can be expressed mathematically as follows:

$$Q = \frac{FD(t_{\max SD})}{FD(t_{\max FD})} \qquad (1)$$

where FD is the first derivative of the PWS with respect to time, SD is the second derivative of the PWS with respect to time, $FD(t_{\max SD})$ is the value of the FD at time $t_{\max SD}$, where $t_{\max SD}$ is the time at which SD has a maximum value (i.e. the a point), and $FD(t_{\max FD})$ is the value of the FD at time $t_{\max FD}$, where $t_{\max FD}$ is the time at which FD has a maximum value.

As noted above, experiments have shown that the quality value Q equals 0.5 in an ideal measurement situation, i.e. where the quality of the obtained PWS is relatively high, or high. Thus, a derived quality value Q indicates the quality of the PWS. In some embodiments, the quality value Q can be compared to a target value T to evaluate the quality of the PWS. In some embodiments the target value T is 0.5, although in other embodiments the target value T can be fine-tuned or adjusted for a specific subject.

Since each cardiac cycle in the PWS will have a respective a point, the maximum of SD can be determined in a portion of the PWS corresponding to a single cardiac cycle of the subject. That is, a portion/window of the PWS can be evaluated that has a duration equal to, or approximately equal to, a cardiac cycle of the subject, and the value of $FD(t_{\max SD})$ in that signal portion can be used in equation (1) to determine the quality value Q. In these embodiments, the value of $FD(t_{\max FD})$ can be based on the maximum of the FD in that signal portion of the PWS, or on the (global) maximum of the full FD. In these embodiments, the quality value Q will relate to the quality of a particular portion of the PWS (i.e. the windowed portion), in which case quality values Q can be determined for different portions of the PWS, e.g. by sliding the window along the PWS.

In some embodiments, a quality indicator QI can be determined from the quality value Q and the target value T according to equation (2) below.

$$QI = e^{-\left(\frac{Q-T}{senstivity}\right)^2} \qquad (2)$$

where the factor 'sensitivity' determines how close the quality value Q needs to be to the target value T in order for the PWS to be considered 'good' quality. According to equation (2), a QI value of 1 will indicate a high quality PWS. As noted above, the target value T in equation (2) can be 0.5, although in other embodiments the target value T can be determined or fine-tuned for a specific subject.

In embodiments where multiple quality values Q or multiple QI are determined for respective portions of a PWS, the quality values Q or quality indicators QI can be averaged to determine an average quality value $Q_{av}$, or average quality indicator $QI_{av}$. This averaging smooths the derived quality values Q or quality indicators QI over a plurality of cardiac cycles. Alternatively, a different smoothing function can be used to smooth the quality values Q or quality indicators *QI* over a number of cardiac cycles. Equation (3) below shows one example of a smoothing function for smoothing the quality indicator *QI* over different cardiac cycles denoted by index *k*. The smoothed quality indicator $QI_{sm}$ is given by:

$$QI_{sm}(k) = \beta\, QI_{sm}(k-1) + (1-\beta)\, QI(k) \qquad (3)$$

where $\beta$ is a smoothing factor. In some embodiments, the smoothing factor has a value of 0.75.

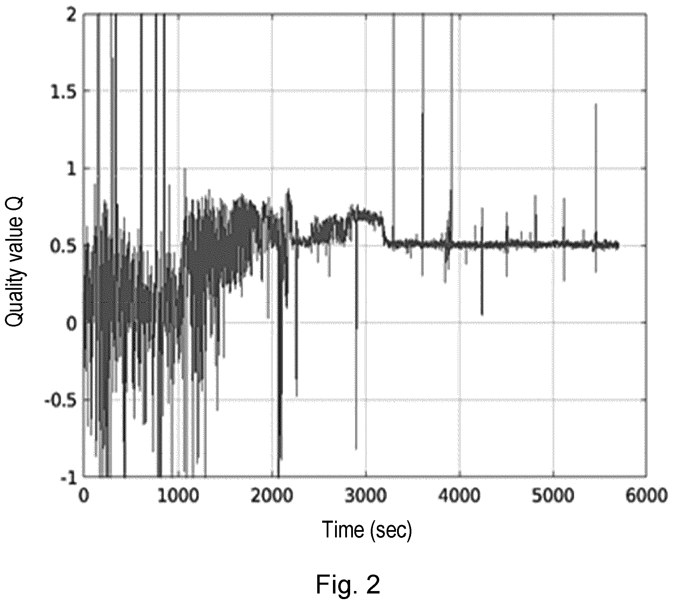

The graph in Fig. 2 illustrates quality values *Q* for a patient over time derived according to equation (1) above. The PWS is a PPG signal, and is obtained from an acute patient, where during the first half of the time period covered by the PPG signal there are signal artefacts due to motion of the patient/PPG sensor and poor perfusion at the periphery (such as the finger where the PPG sensor is attached to the patient). In the second half of the time period, intermittently (every 7 minutes) a cuff is inflated that stops blood flow to the PPG measurement site, resulting in a temporary 'small signal' or 'zero signal'. A quality value *Q* is determined for each measured cardiac cycle. It can be seen that the quality value *Q* is relatively stable at 0.5 in the second half of the time period (apart from the times at which the zero signal occurs), but highly unstable in the first half of the time period.

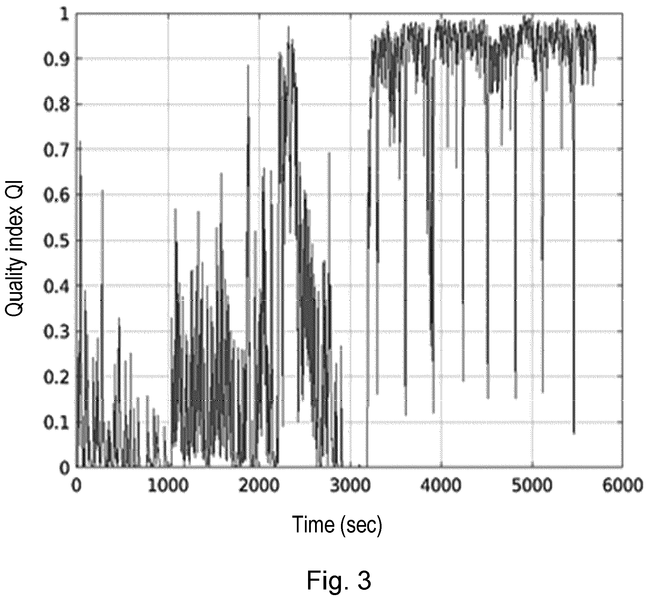

The graph in Fig. 3 illustrates a smoothed *QI* for the patient shown in Fig. 2. The smoothed *QI* is determined according to equations (2) and (3) above, with a value of 0.5 for the target value *T*, a sensitivity of 0.1 and a smoothing factor $\beta$ of 0.75. Similar to Fig. 2, the quality indicator *QI* is relatively stable in the range 0.9 to 1 in the second half of the time period (apart from the times at which the small/zero signal occurs), but highly unstable in the first half of the time period.

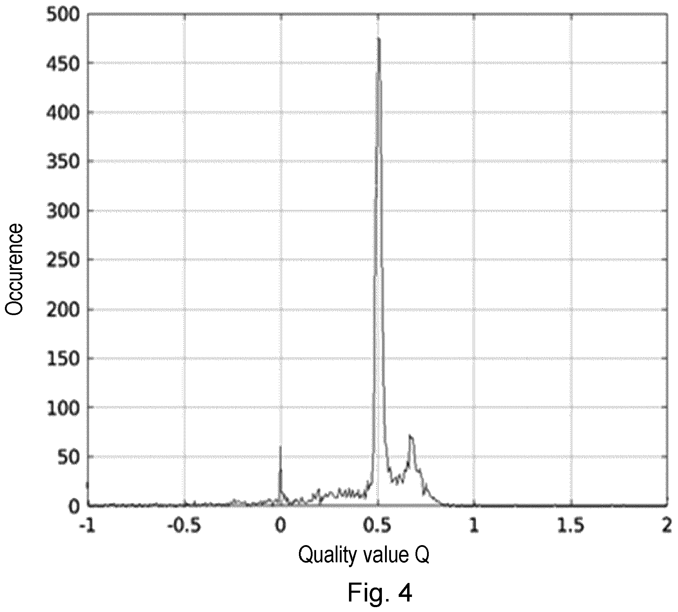

The graph in Fig. 4 shows a distribution (histogram) of the quality values *Q* in Fig. 2. For this patient, it can be seen that the quality values *Q* in Fig. 2 have the highest count (frequency) for exactly 0.5. There is also a second peak visible at around *Q* = 0.7 which is due to the perfusion of the patient being sub-optimal, leading to a poor quality a-PPG computation, and subsequently poor peak detections.

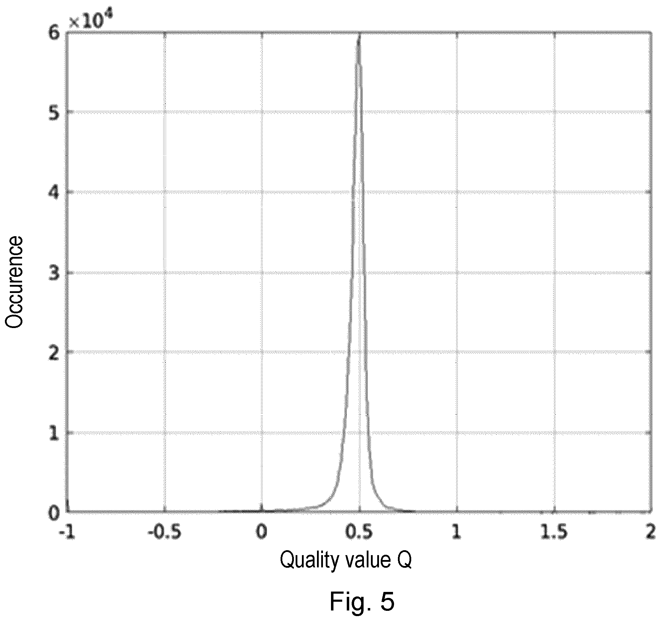

The graph in Fig. 5 shows a similar distribution (histogram) of the quality values *Q* for a dataset including a number of different patients. In particular, Fig. 5 shows a distribution of quality values *Q* derived from a PPG signal for 40 patient cases. In each case, the PPG signal covered a time period of approximately 5 hours. It can be seen that the quality value *Q* has the highest count for exactly 0.5, with the 'tails' of the distribution spanning from 0.4 to 0.6.

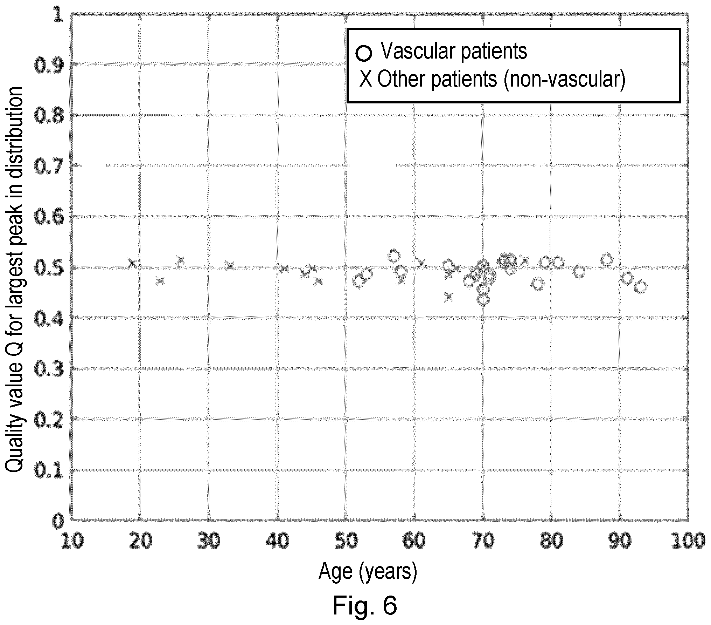

Using the same dataset, the graph in Fig. 6 plots the quality value, *Q*, having the highest frequency/count for each patient against the age of the patient. The graph also distinguishes between patients with vascular issues (the circular data points) and patients with other, non-vascular, issues (the cross data points). Thus, this graph can show whether the quality value *Q* varies with age and/or vascular condition. It can be seen that the quality value *Q* is stable across the patient group, despite a large range in patient ages, and thus a range of arterial compliances. Therefore, Fig. 6 suggests that the quality value *Q* is not particularly dependent on the age of the subject or whether the subject has vascular complications. This makes the quality value *Q* suitable for evaluating the quality of the PWS, including computing the quality indicator *QI,* with an assumption of the optimal quality value *Q* (e.g. 0.5), or target value *T* (e.g. 0.5).

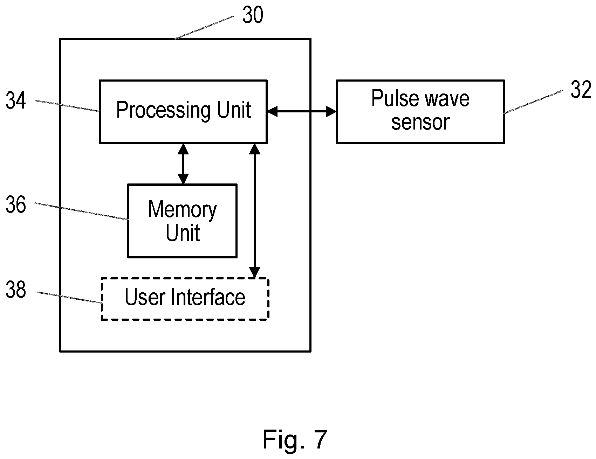

Fig. 7 is a block diagram of an apparatus 30 for analysing a PWS according to various embodiments of the techniques described herein. A pulse wave sensor 32 is shown in Fig. 7 that is used to measure pressure waves at a single point on the body of a subject and to output a PWS. The pulse wave sensor 32 can be a PPG sensor, a pressure sensor, a tonometry-based sensor or a hydraulic sensor pad that can be applied with some application pressure to the (upper) arm of the subject and using a pressure sensor of any type, e.g. the MPXV6115 Series Integrated Silicon Pressure Sensor from NXP Semiconductors. In some embodiments, the pulse wave sensor 32 can be part of, or integral with, the apparatus 30. In other embodiments, the apparatus 30 can be connected to the pulse wave sensor 32, either directly (e.g. wired) or indirectly (e.g. using a wireless communication technology such as Bluetooth, WiFi, a cellular communication protocol, etc.). In alternative embodiments, the apparatus 30 may not be connected to the pulse wave sensor 32, and instead the apparatus 30 can obtain the PWS from another device or apparatus, such as a server or database.

As is known, a PPG sensor 32 can be placed on the body of the subject, for example on an arm, leg, earlobe, finger, etc., can provide an output signal (a 'PPG signal') that is related to the volume of blood passing through that part of the body. The volume of blood passing through that part of the body is related to the pressure of the blood in that part of the body that varies dynamically within each heart cycle. A PPG sensor 32 typically comprises a light sensor, and one or more light sources. The PPG signal output by the PPG sensor 32 may be a raw measurement signal from the light sensor (e.g. the PPG signal can be a signal representing light intensity over time). Alternatively, the PPG sensor 32 may perform some pre-processing of the light intensity signal, for example to reduce noise and/or compensate for motion artefacts, but it will be appreciated that this pre-processing is not required for the implementation of the techniques described herein.

The apparatus 30 may be in the form of, or be part of, a computing device, such as a server, desktop computer, laptop, tablet computer, smartphone, smartwatch, etc., or a type of device typically found in a clinical environment, such as a patient monitoring device (e.g. a monitoring device located at the bedside of a patient in a clinical environment) that is used to monitor (and optionally display) various physiological characteristics of a subject/patient.

The apparatus 30 includes a processing unit 34 that controls the operation of the apparatus 30 and that can be configured to execute or perform the methods described herein to analyse the PWS. The processing unit 34 can be implemented in numerous ways, with software and/or hardware, to perform the various functions described herein. The processing unit 34 may comprise one or more microprocessors or digital signal processors (DSPs) that may be programmed using software or computer program code to perform the required functions and/or to control components of the processing unit 34 to effect the required functions. The processing unit 34 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), field-programmable gate arrays (FPGAs), hardware for implementing a neural network and/or so-called artificial intelligence (AI) hardware accelerators (i.e. a processor(s) or other hardware specifically designed for AI applications that can be used alongside a main processor).

The processing unit 34 is connected to a memory unit 36 that can store data, information and/or signals for use by the processing unit 34 in controlling the operation of the apparatus 30 and/or in executing or performing the methods described herein. In some implementations the memory unit 36 stores computer-readable code that can be executed by the processing unit 34 so that the processing unit 34 performs one or more functions, including the methods described herein. In particular embodiments, the program code can be in the form of an application for a smartwatch, smartphone, tablet, laptop or computer. The memory unit 36 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM) and electrically erasable PROM (EEPROM), and the memory unit 36 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments, the apparatus 30 comprises a user interface 38 that includes one or more components that enables a user of apparatus 30 to input information, data and/or commands into the apparatus 30, and/or enables the apparatus 30 to output information or data to the user of the apparatus 30. Information that can be output by the user interface 38 can include an indication of the quality of the PWS. The user interface 38 can comprise any suitable input component(s), including but not limited to a keyboard, keypad, one or more buttons, switches or dials, a mouse, a track pad, a touchscreen, a stylus, a camera, a microphone, etc., and/or the user interface 38 can comprise any suitable output component(s), including but not limited to a display screen, one or more lights or light elements, one or more loudspeakers, a vibrating element, etc.

Although not shown in Fig. 7, the apparatus 30 may comprise one or more additional sensors for measuring physiological characteristics of a subject, or the processing unit 34 can be configured to receive measurement signals from one or more additional sensors.

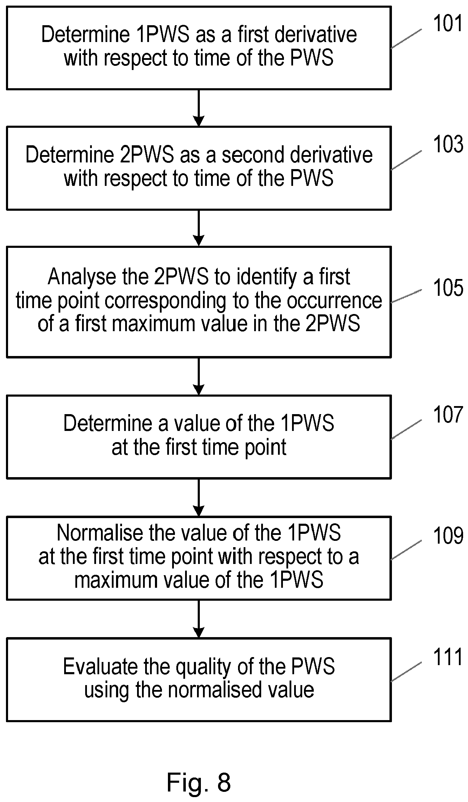

It will be appreciated that a practical implementation of an apparatus 30 may include additional components to those shown in Fig. 7. For example the apparatus 30 may also include a power supply, such as a battery, or components for enabling the apparatus 30 to be connected to a mains power supply. The apparatus 30 may also include interface circuitry for enabling a data connection to and/or data exchange with other devices, including the pulse wave sensor 32 (in embodiments where the pulse wave sensor 32 is separate from the apparatus 30), servers, databases, user devices and/or other sensors. The flow chart in Fig. 8 shows an exemplary method for analysing a PWS obtained from a subject to evaluate the quality of the PWS. In some implementations, the processing unit 34 in the apparatus 30 can be configured to implement the method in Fig. 8. In other implementations computer readable code can be provided that causes a computer or the processing unit 34 to perform the method of Fig. 8 when the computer or processing unit 34 executes the code.

The PWS is received from a pulse wave sensor 32 located at a single measurement point on the subject and the PWS represents pulse wave measurements for a plurality of cardiac cycles (i.e. heart beats) of the subject. The PWS may be received directly from the pulse wave sensor 32, for example as the PWS is generated, and the PWS analysed in real time, or near-real time. Alternatively, the PWS may be stored in a memory, e.g. in memory unit 36 in the apparatus 30 or in a memory unit in a remote device or server, and the PWS retrieved or obtained from the memory when the PWS is to be analysed.

The PWS may be a PPG signal, but the method is also applicable to other forms of PWS. In some implementations, the method in Fig. 8 can be performed periodically or continuously on a PWS as it is received or measured from the subject. In some implementations, the method of Fig. 8 can operate on a windowed portion of a longer PWS, for example a 1-minute window of a PWS covering a time period of 1 hour. However, it should be appreciated that the method can be applied to a PWS having any desired length that covers any number of cardiac cycles. In the following description, references to operations or steps being performed on the PWS relates to performing those operations or steps on the part of the PWS of interest, e.g. a part corresponding to a 1-minute time period.

In a first step of the method, step 101, the first derivative with respect to time of the PWS is determined. This provides the first derivative PWS, denoted 1PWS. As noted above, this step may operate on the complete PWS, or a portion of the PWS.

In another step of the method, step 103, the second derivative with respect to time of the PWS is determined. This provides the second derivative PWS, denoted 2PWS. It will be appreciated that steps 101 and 103 can be performed in either order, or at the same time.

In step 105, the 2PWS is analysed to identify a first time point corresponding to the occurrence of a first maximum value in the 2PWS. Step 105 aims to identify an 'a' reference point in the 2PWS, i.e. the start of the direct pulse wave. As described above with reference to Fig. 1, each cardiac cycle in an 'acceleration waveform' (the second derivative of the PWS with respect to time) can be considered to comprise five 'fiducial points' or 'reference points'. The *a* reference point marks the start of the direct pulse wave, which is the onset of the pulse wave (i.e. the pulse of blood caused by the beat of the heart) and the *b* point marks the end of the direct pulse wave. The *e* point marks the end of the systolic period (aortic valve closure), and for the purposes of this disclosure it is assumed that the c point marks the start of the reflected wave and the d point marks the end of the reflected wave. Thus, step 105 aims to identify an 'a' reference point in the 2PWS, i.e. the start/onset of the direct pulse wave. Step 105 can use conventional peak detection techniques to identify the maximum value in the 2PWS. The first time point can be determined as the time point corresponding to the sample with the first maximum value. Alternatively, the maximum value of the 2PWS can be determined from a plurality of sample values at or around the maximum value. For example, multiple samples can be used in an interpolation technique, for example cubic-interpolation, to get improved accuracy in the determination of the maximum value of the 2PWS in case the PWS signal does not have a sufficiently high sampling rate.

In some embodiments, the quality of the PWS is to be evaluated on a cardiac cycle-by-cycle basis, so step 105 operates on a portion of the 2PWS, with the portion having a duration equal to or less than the duration of a cardiac cycle. In this way, the maximum of the 2PWS in the portion should correspond to the 'a' reference point. The duration of the cardiac cycle (and thus the duration of the portion) can be predetermined, e.g. set based on a standard cardiac cycle duration, such as 1 second (for a pulse rate of 60 beats per minute, bpm), or determined by analysing the PWS to determine the pulse rate. In the latter case, conventional PWS analysis techniques can be used to determine the pulse rate from the PWS, and thus determine the duration of the portion to analyse in step 105.

In step 107, a value of the 1PWS is determined at the first time point. That is, the value of the 1PWS is determined at the time that the 2PWS is at the first maximum value. This step can comprise simply determining the value of the 1PWS at the first time point, or the value of the 1PWS closest to the first time point in the event that there is no sample value for the 1PWS at the first time point. Alternatively, the value of the 1PWS at the first time point can be determined from a plurality of sample values at or around the first time point. For example, the value of the 1PWS can be determined as a function of (e.g. a linearly weighted average) of two values of the 1PWS at or around the first time point. This embodiment is useful in case the PWS (and thus the 1PWS) does not have a sufficiently high sampling rate and will mitigate jitter in the determination of the sample values of the 1PWS signal.

In step 109, the value of the 1PWS determined in step 107 is normalised with respect to a maximum value of the 1PWS. This normalised value is the quality value Q described above. Step 109 can therefore comprise determining the maximum value of the 1PWS, and normalising the value of the 1PWS at the first time point according to the determined maximum value (e.g. by dividing by the determined maximum value according to equation (1)). Step 109 may comprise determining the global maximum value of the 1PWS, or, in embodiments where step 105 is performed for a portion of the 2PWS (e.g. a portion having a duration equal to or less than a cardiac cycle), step 109 can comprise determining the maximum value of the 1PWS in the portion evaluated in step 105. Conventional peak detection techniques can be used to identify the maximum value in the 1PWS. The maximum value of the 1PWS can be determined as the highest value of any sample in the 1PWS (or the portion of the 1PWS, if applicable). Alternatively, the maximum value of the 1PWS can be determined from a plurality of sample values at or around the maximum value. For example, multiple samples can be used in an interpolation technique, for example cubic-interpolation, to get improved accuracy in the determination of the maximum value of the 1PWS in case the PWS signal does not have a sufficiently high sampling rate. Either of these embodiments are useful in case the maximum of the 1PWS is a result of a signal artefact.

In step 111, the quality of the PWS is evaluated using the normalised value. In some embodiments, the normalised value (e.g. the quality value Q) indicates the quality of the PWS. In some embodiments, the quality of the PWS is indicated by a difference between the normalised value and a target value, T. The target value, T, may be a static value, e.g. 0.5, or a value that depends on one or more characteristics of the subject, such as age and/or vascular condition of the subject.

In some embodiments, in step 111 the quality of the PWS can be evaluated using a quality indicator *QI* determined according to equation (2) above.

In some embodiments, multiple normalised values can be determined for different cardiac cycles in the PWS, and these multiple normalised values used in step 111 to evaluate the quality of the PWS. Thus, for example, the method can further comprise repeating steps 105-109 for a second time point corresponding to the occurrence of a second maximum value in the 2PWS. This second maximum value will relate to a different cardiac cycle to the first maximum value. Steps 105-109 can be repeated any desired number of times. In these embodiments, the quality of the PWS can be evaluated based on the multiple normalised values themselves, or based on respective quality indicators $QI$ determined according to equation (2) above. The quality may be evaluated based on a function of the normalised values, such as an average of the normalised values or an average of the quality indicators $QI$ determined from the normalised values. In either case, the function may be smoothing function, for example as defined in equation (3) above.

As noted above, in some embodiments the sampling rate of the PWS can be increased. Thus, prior to step 101, the method can further comprise obtaining a sensor signal from a pulse wave sensor that comprises pulse wave measurements according to a first sampling rate; and generating the PWS by using an interpolation technique on the sensor signal to increase the sampling rate.

In some embodiments the method further comprises a step in which it is determined whether to compute a physiological characteristic for the subject from the PWS according to the quality of the PWS. For example, if the quality of the PWS is too low, or below some threshold value, then the PWS, or that part of the PWS/cardiac cycle to which the normalised value(s) relate, may not be used to compute a physiological characteristic. On the other hand, if the quality of the PWS is sufficient, e.g. above some threshold value, then the PWS, or that part of the PWS/cardiac cycle to which the normalised value(s) relate, may be used to compute a physiological characteristic.

In some embodiments, the method can further comprise outputting an indication of the quality or reliability of the PWS according to the evaluated quality. The indication of the quality or reliability can be output via the user interface 38.

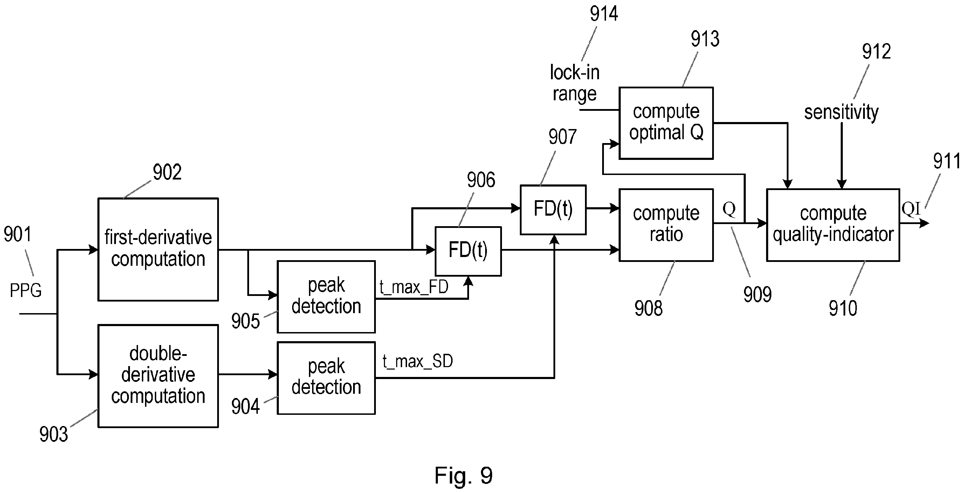

Fig. 9 is a functional block diagram illustrating various operations in analysing a PWS according to various exemplary embodiments. The various operations and functions shown in Fig. 9 can be implemented by the processing unit 34 shown in Fig. 7. In this exemplary illustrated embodiment, the PWS is a PPG signal 901. The PPG signal 901 is input to a First Derivative Computation block 902 that determines the first order derivative of the PPG signal with respect to time (1PPG) and a Second Derivative Computation block 903 that determines the second order derivative of the PPG signal with respect to time (2PPG). It will be appreciated that in an alternative implementation, the Second Derivative Computation block 903 can be replaced by another First Derivative Computation block that operates on the 1PPG output by the First Derivative Computation block 902 to determine the 2PPG.

In some embodiments, although not shown in Fig. 9, a Serial/parallel (S/P) Converter can be provided which splits or divides the PPG signal 901 into one or more windows of $N_x$ samples, before inputting the windowed PPG signal to the First Derivative Computation block 902 and the Second Derivative Computation block 903. The windows of the PPG signal may overlap or be contiguous.

The 2PPG is input to a Peak Detection block 904 that detects the first maximum value of the 2PPG (according to step 105 above). The Peak Detection block 904 outputs the first time point, $t_{maxSD}$, which is the time at which the first maximum value occurred.

The 1PPG is input to another Peak Detection block 905, First Value Extraction block 906 (denoted $FD(t)$) and Second Value Extraction block 907 (also denoted $FD(t)$). The Peak Detection block 905 detects the maximum value of the 1PPG (that is used in step 109 above) and outputs the time at which this maximum value occurs, denoted $t_{maxFD}$, to the First Value Extraction block 906.

The First Value Extraction block 906 outputs the value of the 1PPG at the time $t_{maxFD}$. It will be appreciated that the First Value Extraction block 906 can be omitted if the Peak Detection block 905 outputs the maximum value of the 1PPG itself rather than outputting the time at which the maximum value occurs.

The Second Value Extraction block 907 receives the 1PPG signal and receives $t_{maxSD}$ from the Peak Detection block 904. The Second Value Extraction block 907 outputs the value of the 1PPG at the time $t_{maxSD}$ (the time at which the maximum value occurred in the 2PPG).

The values of the 1PPG determined by the First and Second Value Extraction blocks 906, 907 are input to a Ratio Computation block 908, which calculates the quality value $Q$ 909 by dividing the value of the 1PPG signal at the time that the 2PPG signal has a maximum by the maximum value of the 1PPG signal. Ratio Computation block 908 implements step 109 above.

This quality value $Q$ 909 can be used to evaluate the quality of the PPG signal (as in step 111 above). In some embodiments, the quality value $Q$ is compared to a target value T that represents a high quality PPG signal.

However, in further embodiments, a quality indicator $QI$ can be computed from the quality value $Q$ 909 by a Quality Indicator Computation block 910. The Quality Indicator Computation block 910 compares the quality value $Q$ 909 to a target value $T$ (which is denoted in Fig. 9 as an 'optimal $Q$') to determine the quality indicator $QI$ 911. The Quality Indicator Computation block 910 can determine the quality indicator $QI$ 911 according to equation (2) above, in which case the Quality Indicator Computation block 910 can also receive a sensitivity value 912. In some embodiments the optimal $Q$ value is predetermined (e.g. 0.5), but in other embodiments the optimal $Q$ value can be computed by an Optimal $Q$ Computation block 913 and output to the Quality Indicator Computation block 910. This may be useful as some subjects may have an optimal *Q* value that is slightly below 0.5 or slightly above 0.5, as suggested by Fig. 6. The quality value *Q* 909 can be input to the Optimal *Q* Computation block 913, along with a lock-in range 914 which restricts the range of values of the quality value *Q* used to determine optimal *Q* to values in the lock-in range. This acts to restrict the possible values of optimal *Q* to the particular range of values. For example, the lock-in range can be between 0.4 and 0.6. The Optimal *Q* Computation block 913 may compute the optimal *Q* by determining a long term average of the quality values *Q* for the subject. The calculation of the long term average of the quality value *Q* may be stopped, or the current quality value *Q* discarded from consideration in determining the optimal *Q,* if the current value of the quality value *Q* is outside the lock-in range.

Therefore, there is provided techniques for the improved analysis of the quality of a PWS. Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1.  A computer-implemented method for analysing a pulse wave signal, PWS, obtained from a subject, wherein the PWS comprises pulse wave measurements for one or more cardiac cycles of the subject during a first time period, the method comprising:

    (i) determining (101) 1PWS as a first derivative with respect to time of the PWS;
    (ii) determining (103) 2PWS as a second derivative with respect to time of the PWS;
    (iii) analysing (105) the 2PWS to identify a first time point corresponding to the occurrence of a first maximum value in the 2PWS;
    (iv) determining (107) a value of the 1PWS at the first time point;
    (v) normalising (109) the value of the 1PWS at the first time point with respect to a maximum value of the 1PWS;
    (vi) evaluating (111) the quality of the PWS using the normalised value.

2.  A method as claimed in claim 1, wherein the first maximum value in the 2PWS and/or the value of the 1PWS at the first time point and/or the maximum value of the 1PWS are determined from a plurality of sample values of the 2PWS and/or 1PWS.

3.  A method as claimed in claim 1 or 2, wherein step (vi) comprises:
    determining the quality of the PWS according to a difference between the normalised value and a target value, T.

4.  A method as claimed in any of claims 1-3, wherein step (vi) comprises:
    determining a quality indicator, QI, according to:

$$QI = e^{-\left(\frac{Q-T}{s}\right)^2}$$

    where Q is the normalised value and s is a sensitivity value representing acceptable deviation of Q from the target value, T.

5.  A method as claimed in any of claims 1-4, wherein the method further comprises:

    performing at least one repetition of steps (iii)-(v) for at least a second time point corresponding to the occurrence of at least a second maximum value in the 2PWS; and
    wherein step (vi) comprises evaluating (111) the quality of the PWS using the normalised values.

6.  A method as claimed in claim 5, wherein step (vi) comprises evaluating (111) the quality of the PWS using a function of the normalised values.

7.  A method as claimed in any of claims 1-6, wherein the method further comprises:
    determining whether to compute a physiological characteristic for the subject from the PWS according to the quality of the PWS.

8.  A computer program product comprising a computer readable medium having computer readable code embodied therein, the computer readable code being configured such that, on execution by a suitable computer or processor, the computer or processor is caused to perform the method of any of claims 1-7.

9.  An apparatus (30) configured to analyse a pulse wave signal, PWS, obtained from a subject, wherein the PWS comprises pulse wave measurements for one or more cardiac cycles of the subject during a first time period, the apparatus (30) comprising a processing unit (34) configured to:

    (i) determine 1PWS as a first derivative with respect to time of the PWS;
    (ii) determine 2PWS as a second derivative with respect to time of the PWS;
    (iii) analyse the 2PWS to identify a first time point corresponding to the occurrence of a first maximum value in the 2PWS;
    (iv) determine a value of the 1PWS at the first time point;
    (v) normalise the value of the 1PWS at the first time point with respect to a maximum value of the 1PWS;
    (vi) evaluate the quality of the PWS using the normalised value.

10. An apparatus (30) as claimed in claim 9, wherein the first maximum value in the 2PWS and/or the value of the 1PWS at the first time point and/or the maximum value of the 1PWS are determined from a plurality of sample values of the 2PWS and/or 1PWS.

11. An apparatus (30) as claimed in claim 9 or 10, wherein in operation (vi) the processing unit (34) is configured to:
    determine the quality of the PWS according to a difference between the normalised value and a target value, T.

12. An apparatus (30) as claimed in any of claims 9-11, wherein in operation (vi) the processing unit (34) is configured to:
    determine a quality indicator, QI, according to:

$$QI = e^{-\left(\frac{Q-T}{s}\right)^2}$$

    where Q is the normalised value and s is a sensitivity value representing acceptable deviation of Q from the target value, T.

13. An apparatus (30) as claimed in any of claims 9-12, wherein the processing unit (34) is further configured to:

    perform at least one repetition of operations (iii)-(v) for at least a second time point corresponding to the occurrence of at least a second maximum value in the 2PWS; and
    wherein in operation (vi) the processing unit (34) is configured to evaluate the quality of the PWS using the normalised values.

14. An apparatus (30) as claimed in claim 13, wherein in operation (vi) the processing unit (34) is configured to evaluate the quality of the PWS using a function of the normalised values.

15. An apparatus (30) as claimed in any of claims 9-14, wherein the processing unit (34) is further configured to:
    determine whether to compute a physiological characteristic for the subject from the PWS according to the quality of the PWS.

Fig.1

EP 4 094 682 A1

Fig. 2

Fig. 3

Fig. 4

Fig. 5

Fig. 6

Fig. 7

Determine 1PWS as a first derivative
with respect to time of the PWS
— 101

Determine 2PWS as a second derivative
with respect to time of the PWS
— 103

Analyse the 2PWS to identify a first
time point corresponding to the occurrence
of a first maximum value in the 2PWS
— 105

Determine a value of the 1PWS
at the first time point
— 107

Normalise the value of the 1PWS
at the first time point with respect to a
maximum value of the 1PWS
— 109

Evaluate the quality of the PWS
using the normalised value
— 111

Fig. 8

Fig. 9

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/360984 A1 (ALBADAWI HAITHEM [US] ET AL) 15 December 2016 (2016-12-15) <br><br> * paragraphs [0017], [0018], [0026], [0029], [0034] - [0038], [0042], [0052], [0066] - [0072] * <br>----- | 1,2, 4-10, 12-15 | INV. A61B5/021 A61B5/00 A61B5/024 |
| X | US 2017/065230 A1 (SINHA TUHIN [US] ET AL) 9 March 2017 (2017-03-09) * paragraphs [0019], [0029], [0034], [0056], [0087] - [0093] * <br>----- | 1-3,8-11 | |
| X | CN 112 545 472 A (CHENGDU CVHEALTH SCIENCE AND TECH CO LTD) 26 March 2021 (2021-03-26) * paragraphs [0054] - [0087] * <br>----- | 1,9 | |

TECHNICAL FIELDS
SEARCHED (IPC)

A61B

The present search report has been drawn up for all claims

| Place of search | Date of completion of the search | Examiner |
|---|---|---|
| The Hague | 12 November 2021 | Kowalczyk, Szczepan |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.....................................................................................................

& : member of the same patent family, corresponding
document

EPO FORM 1503 03.82 (P04C01)

# EP 4 094 682 A1

## ANNEX TO THE EUROPEAN SEARCH REPORT
## ON EUROPEAN PATENT APPLICATION NO.

EP 21 17 6015

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2021

| Patent document cited in search report | | Publication date | Patent family member(s) | | Publication date |
|---|---|---|---|---|---|
| US 2016360984 | A1 | 15-12-2016 | CN 107771052 A | | 06-03-2018 |
| | | | EP 3307139 A1 | | 18-04-2018 |
| | | | US 2016360984 A1 | | 15-12-2016 |
| | | | WO 2016205095 A1 | | 22-12-2016 |
| US 2017065230 | A1 | 09-03-2017 | US 2017065230 A1 | | 09-03-2017 |
| | | | US 2019357855 A1 | | 28-11-2019 |
| CN 112545472 | A | 26-03-2021 | NONE | | |

For more details about this annex : see Official Journal of the European Patent Office, No. 12/82

22

**REFERENCES CITED IN THE DESCRIPTION**

**Non-patent literature cited in the description**

- **ELYAS SABETI.** Signal quality measure for pulsatile physiological signals using morphological features: Applications in reliability measure for pulse oximetry. *Informatics in Medicine Unlocked,* 2019, vol. 16